Europäisches Patentamt

European Patent Office

Office européen des brevets

(19)

(11) EP 0 659 721 B1

(12) **EUROPÄISCHE PATENTSCHRIFT**

(45) Veröffentlichungstag und Bekanntmachung des Hinweises auf die Patenterteilung:
**10.06.1998 Patentblatt 1998/24**

(51) Int Cl.[6]: **C07C 29/50**, C07C 29/17, C07D 309/04, C07D 309/18

(21) Anmeldenummer: **94120086.7**

(22) Anmeldetag: **19.12.1994**

(54) **Verfahren zur Photooxidation von Terpenolefinen**

Method for the photooxidation of terpene olefins

Procédé pour la photooxydation des oléfines térpéniques

(84) Benannte Vertragsstaaten:
**CH DE ES FR LI**

(30) Priorität: **23.12.1993 DE 4344163**

(43) Veröffentlichungstag der Anmeldung:
**28.06.1995 Patentblatt 1995/26**

(73) Patentinhaber: **HAARMANN & REIMER GMBH**
**D-37601 Holzminden (DE)**

(72) Erfinder:
- **Scharf, Professor Dr. Hans-Dieter**
  **D-52159 Roetgen (DE)**
- **Esser, Dr. Peter**
  **D-37603 Holzminden (DE)**
- **Kuhn, Dr. Walter**
  **D-37603 Holzminden (DE)**
- **Pelzer, Dr. Ralf**
  **D-37699 Fürstenberg (DE)**

(74) Vertreter: **Petrovicki, Wolfgang, Dr. et al**
**Bayer AG**
**Konzernbereich RP**
**Patente und Lizenzen**
**51368 Leverkusen (DE)**

(56) Entgegenhaltungen:
**DE-B- 1 235 306          DE-C- 933 925**

- **LIEBIGS ANNALEN DER CHEMIE, Bd. 584, 1953 WEINHEIM, DE, Seiten 177-198, G.O. SCHENCK, ET AL.: 'Photochemische Reaktionen III. Über die Bildung von Hydroperoxyden bei photosensibilisierten Reaktionen von O2 mit geeigneten Akzeptoren, insbesondere mit alpha- und beta-Pinen'**

- **ANGEWANDTE CHEMIE, Bd. 73,Nr. 16, 1961 WEINHEIM, DE, Seite 578 G. OHLOFF, ET AL.: 'Darstellung con "Rosenoxyden" und anderen Hydropyran-Derivaten über Photohydroperoxyden'**
- **CHEMISTRY AND INDUSTRY, Nr. 10, 10.März 1962 LONDON, GB, Seite 459 G.O. SCHENCK, ET AL.: 'Photosensitised oxidation of (+)-delta3-carene'**
- **CHEMISCHE BERICHTE Bd. 98, Nr. 9, 1965, WEINHEIM, DE, Seiten 3045 - 3049 E. KLEIN, ET AL.: 'Die photosensibilisierte O2-Übertragung auf (+)-alpha-Thujen'**
- **HELVETICA CHIMICA ACTA, Bd. 47,Nr. 2, 1964 BASEL, CH, Seiten 602-626, G. OHLOFF, ET AL.: 'Zur Darstellung von Tetrahydropyran- und Tetrahydrofuranderivaten aus 1,7- bzw. 1,6-Allyldiolen durch Deyhdration in der Allylstellung'**
- **CHEMICAL ABSTRACTS, vol. 98, no. 25, 20.Juni 1983 Columbus, Ohio, US; abstract no. 215833h, Seite 581; & ES-A-505 634 (LUCTA)**
- **TETRAHEDRON, Bd. 21,Nr. 8, August 1965 OXFORD, GB, Seiten 2173-2178, E. KLEIN, ET AL.: 'Die photosensibilierten Oxydation semicyclischer Doppelbindungen'**
- **PATENT ABSTRACTS OF JAPAN vol. 5 no. 140 (C-070) ,4.September 1981 & JP-A-56 075472 (LION CORPORATION) 22.Juni 1981,**
- **PATENT ABSTRACTS OF JAPAN vol. 4 no. 61 (C-009) ,8.Mai 1980 & JP-A-55 028965 (LION CORPORATION) 29.Februar 1980,**
- **La Chimica e L' Industria 64, 156 - 166 (1982)**

**Beschreibung**

Die Erfindung betrifft ein Verfahren zur Oxidation olefinischer Terpene mit Sauerstoff unter Sonnen- oder Kunstlicht hoher Bestrahlungsdichte.

Die Oxidation olefinischer Terpene mit Sauerstoff unter Lichteinwirkung in Gegenwart eines Sensibilisators stellt eine brauchbare Methode zur Herstellung von Peroxiden und Hydroperoxiden dar, die wertvolle Zwischenprodukte für die Herstellung von Riechstoffen sind.

So sind beispielsweise schon $\alpha$- und $\beta$-Pinen (DE-PS 933 925 und G.O. Schenck et al., Liebigs Ann. Chem. 584, 177 (1953)), $\beta$-Citronellol (G.Ohloff et al.. Angew. Chem. 73, 578 (1961)), 3-Caren (G.O. Schenck et al., Chem. and Ind. 1962, 459), $\alpha$-Thujen (E. Klein et al., Chem. Ber. 98, 3045 (1965)), Terpinolen (DE-AS 12 35 306) und Nerol (G.Ohloff et al., Helv. Chim. Acta 47, 602 (1964)) photooxidiert worden. Die Autoren haben teils Sonnenlicht, teils künstliches Licht verwendet; in vielen Fällen war die Ausbeute unbefriedigend, regelmäßig aber die Ausbeute im Hinblick auf die Reaktionszeit unzureichend.

Es wurde nun überraschenderweise gefunden, daß man weniger Nebenprodukte bei höheren Umsätzen erhält, wenn man eine sehr hohe Bestrahlungsstärke wählt.

Gegenstand der Erfindung ist also ein Verfahren zur Oxidation olefinischer Terpene mit Sauerstoff unter Lichteinfluß, dadurch gekennzeichnet, daß nicht mehr als 10 % der Gesamtenergie des emittierten Lichts auf den Wellenlängenbereich von 200 bis 400 nm, bezogen auf den von der Lichtquelle ausgestrahlten Wellenlängenbereich von 200 bis 700 nm, entfallen und die Bestrahlungsstärke so gewählt wird, daß die Reaktion in weniger als 12 Stunden einen Umsatz von 95 % erreicht.

Ein solcher Umsatz wird im allgemeinen bei einer Bestrahlungsstärke von 5 bis 200 Sonnen, vorzugsweise von 10 bis 100 Sonnen erzielt.

Es wurde weiterhin gefunden, daß die Reaktion mit besonders gutem Ergebnis verläuft, wenn UV-Licht weitestgehend ausgeschlossen wird, d.h. daß nicht mehr als 10 % der Gesamtenergie des emittierten Lichts auf den Wellenlängenbereich von 200 bis 400 nm und vorzugsweise nicht mehr als 1 % der Gesamtenergie des emittierten Lichts auf den Wellenlängenbereich von 200 bis 300 nm entfallen - jeweils bezogen auf den von der Lichtquelle ausgestrahlten Wellenlängenbereich von 200 bis 700 nm.

Für das erfindungsgemäße Verfahren bevorzugte olefinische Terpene umfassen beispielsweise acyclische, gegebenenfalls Hydroxylgruppen - enthaltende olefinische Terpene wie Myrcen, Ocimen, Geraniol, Nerol, Linalool, Myrcenol, Lavandulol, Citronellol, monocyclische olefinische Terpene wie Limonen, $\alpha$- und $\gamma$-Terpinen, Terpinolen, $\alpha$- und $\beta$-Phellandren und bicyclische olefinische Terpene wie $\alpha$- und $\beta$-Pinen, Camphen,

Caren und $\alpha$-Thujen.

Die Reaktion kann in einem unter Reaktionsbedingungen inerten organischen Lösungsmittel durchgeführt werden. Diese Lösungsmittel sollen Ausgangs- und Endprodukt sowie den Sensibilisator ausreichend lösen und die Lebensdauer von Singulett-Sauerstoff günstig beeinflussen. Solche Lösungsmittel umfassen beispielsweise aliphatische und cycloaliphatische Kohlenwasserstoffe wie Benzin und Cyclohexan, chlorierte aliphatische Kohlenwasserstoffe wie Dichlormethan, Chloroform und Tetrachlormethan, hochfluorierte Kohlenwasserstoffe wie Hexafluorbenzol, Polyfluorheptan, aromatische Kohlenwasserstoffe wie Benzol, Toluol und Xylole, chlorierte aromatische Kohlenwasserstoffe wie Chlorbenzol, ein- und zweiwertige aliphatische Alkohole wie Methanol, Ethanol, n-Propanol, Isopropanol, n- und tert.-Butanol, Ethylenglykol, Ether wie Diethyl- und Dibutylether, Tetrahydrofuran und Dioxan, $C_2$-$C_4$-Carbonsäure-$C_1$-$C_6$-alkylester wie Essigsäureethyl- und -butylester, Ether und Ester von Ethylen- und Propylenglykol wie Ethylenglykolmonomethyl- und -monoethylether, Ethylenglykolmonomethyl- und -monoethyletheracetat und Propylenglykol-diacetat, Ketone wie Aceton, Methylethyl-keton und Cyclohexanon, $C_1$-$C_6$-Carbonsäuren wie Essigsäure, Amide wie Dimethylformamid, Dimethylacetamid, Sulfolan, N-Methylpyrrolidon und N-Methylcaprolactam sowie Nitrile wie Acetonitril. Aliphatische $C_1$-$C_6$-Alkohole werden besonders bevorzugt.

Bevorzugte Sensibilisatoren umfassen Xanthen-Farbstoffe wie Fluorescein, Eosin, Erythrosin B und Bengalrosa, Phenothiazin-Farbstoffe wie Methylenblau und Porphyrine bzw. Porphine wie Tetraphenylporphyrin, Zink- Tetraphenylporphin und Chlorophyll. Die gewählte Konzentration an Sensibilisator hängt im wesentlichen von der Schichtdicke der bestrahlten Lösung am Einstrahlungsfenster ab; sie beträgt im allgemeinen 100 ppm bis 5 Gew.-%, bezogen auf bestrahlte Lösung. Es kann vorteilhaft sein, einen Teil des Sensibilisators im Laufe der Reaktion nachzudosieren.

Zur Oxidation können reiner Sauerstoff oder sauerstoffhaltige Gase wie Luft verwendet werden. Die Gasdosierung erfolgt an der Stelle höchstens Drucks; die Menge orientiert sich an der Löslichkeit im gewählten Lösungsmittel bei Reaktionstemperatur. Die Mischung mit dem Lösungsmittel kann auf übliche Weise, z.B. mit einem statischen Mischer, erfolgen. Um mit hoher Sauerstoffkonzentration arbeiten zu können, kann die Reaktion auch unter Druck (vorzugsweise bis zu 10 bar) ausgeführt werden.

Die zu verwendenden Reaktorsysteme umfassen neben einer Gasdosierung ein Lichtkonzentrierungssystem, ein Gefäß mit Bestrahlungsfenster, eine Transportvorrichtung für die Reaktionslösung (vorzugsweise eine Pumpvorrichtung) und gegebenenfalls eine Kühlvorrichtung.

Als Lichtkonzentrierungssysteme eignen sich z.B. besonders gut Parabolspiegel, beispielsweise in Form von Parabolspiegelrinnen, und Heliostatenfelder oder Paraboldishes. Die Form der Bestrahlungsfenster richtet sich vernünftigerweise nach der Art des verwendeten Lichtkonzentrierungssystems; beispielsweise wird man bei Verwendung einer Parabolspiegelrinne als Reaktor ein in der Brennlinie dieser Spiegelrinne konzentriertes Glasrohr einsetzen. Die Aufnahme thermischer Energie kann man beispielsweise dadurch begrenzen, daß man den Durchgang nicht absorbierter Strahlung durch den Reaktor ermöglicht oder Strahlen mit Wellenlängen oberhalb der Grenzwellenlänge der photochemischen Reaktion durch Einsatz eines Teilerspiegels, der langwelliges Licht reflektiert, aber den Durchtritt kurzwelligen Lichts gestattet, erst gar nicht in die Reaktionslösung gelangen läßt.

Nach einer bevorzugten Ausführungsform wird die Sonne als Lichtquelle benutzt. Der durch das Lichtkonzentrierungssystem erreichte Konzentrierungsgrad ergibt sich als Erhöhung der Bestrahlungsstärke am Bestrahlungsfenster des Photoreaktors in Relation zur direkten solaren Einstrahlung. Diese wird mit Pyrheliometern bestimmt, einem durch das World Radiation Center in Davos/Schweiz standardisierten Verfahren.

Im Falle der Verwendung von Kunstlicht eignen sich z.B. undotierte Quecksilber-Hochdrucklampen, Xenonlampen, vorzugsweise Thalliumjodid-dotierte Quecksilber-Hochdrucklampen und Natrium-Niederdrucklampen. Nach einer bevorzugten Ausführungsform wird man die Lampen parallel um ein Absorberrohr anordnen.

Die Bestimmung des auf den Wellenlängenbereich von 200 bis 400 nm bzw. 200 bis 300 nm entfallenden Anteils der eingestrahlten Gesamtenergie erfolgt am besten durch eine Differenzmessung, wonach man zunächst die bis 700 nm emittierte Gesamtenergie (TE) ermittelt und danach unter Verwendung zusätzlicher geeigneter Filter die zwischen 400 und 700 nm bzw. die zwischen 300 und 700 nm emittierte Teilenergie (PE) der Lichtquelle bestimmt; die Differenz

$$100 - \frac{TE-PE}{TE}$$

ergibt dann den prozentualen Anteil der zwischen 200 und 400 nm bzw. zwischen 200 und 300 nm liegenden Strahlungsenergie.

Das Volumen der mit Bestrahlungsfenster versehenen Reaktorteile pro Gesamtvolumen des Reaktors richtet sich vor allem nach sicherheitstechnischen Belangen und beträgt im allgemeinen 5 bis 50, vorzugsweise 7 bis 30 %.

Die Reaktion wird vorzugsweise bei Temperaturen von 0 bis 60°C, insbesondere von 10 bis 40°C durchgeführt.

Die durch das erfindungsgemäße Verfahren hergestellten Photooxidationsprodukte stellen wertvolle Zwischenprodukte für die Riechstoffindustrie dar. Aufgrund ihrer Hydroperoxidgruppen wird man oft auf eine Aufarbeitung der Reaktionsmischung verzichten und in der Regel die Weiterverarbeitung des Produkts vorziehen. So lassen sich die Hydroperoxide beispielsweise mit üblichen Reduktionsmitteln, wie sie z.B. in "Methoden der Organischen Chemie" (Houben-Weyl), 4. Aufl. Bd. 4/1d, S. 456, Georg Thieme Verlag, Stuttgart-New York 1981, und in den dort genannten Literaturstellen beschrieben sind, in die entsprechenden Alkohole überführen. Bevorzugte Reduktionsmittel umfassen z.B. Wasserstoff in Gegenwart üblicher Hydrierkatalysatoren, wie z.B. Raney-Nickel oder Palladium auf Aktivkohle, Alkalialuminiumhydride wie Lithium- und Natriumaluminiumhydride, Triphenylphosphan und insbesondere Natriumsulfit - vorzugsweise in wäßriger Lösung. Das resultierende Hydroxyterpen kann dann als solches oder in Form seiner Folgeprodukte als Riechstoff eingesetzt werden.

So kann etwa das aus Citronellol entstehende Hydroperoxid zur Hydroxylverbindung reduziert werden, worauf dann ein Ringschluß, z.B. in Gegenwart von Schwefelsäure, zu Rosenoxid angeschlossen werden kann.

Das aus α-Thujen entstehende Hydroperoxid kann zu 4-Hydroxy-β-thujen reduziert und dann, z.B. in Gegenwart von Raney-Nickel, zum Sabinenhydrat hydriert werden.

Das aus Nerol entstehende Hydroperoxid kann zur entsprechenden Hydroxylverbindung reduziert und diese dann säurekatalysiert zu Neroloxid cyclisiert werden.

Das aus Terpinolen entstehende Hydroperoxid kann zu p-Mentha-1.8-dien-4-ol reduziert und dieses katalytisch selektiv zu Terpineol-(4) hydriert werden.

## Beispiel

### Bestrahlung mit Sonnenlicht

Als Reaktor diente ein Röhrenreaktor (Volumen 70 l) mit geschlossenem Kreislauf bestehend aus einer schwenkbaren Parabolspiegelrinne (Höhe 4,5 m, Fläche 7,3 m², Aluminium-bedampftes Polytetrafluorethylen, rückseitig mit Polyesterfolie versiegelt), einem in deren Brennlinie angeordneten Absorberrohr aus Glas und einem Versorgungsteil enthaltend Vorratsgefäß, Pumpe, Wärmetauscher, Gasdosierungsvorrichtung und statischen Mischer. Die Parabolspiegelrinne wurde dem Sonnenstand nachgeführt. Die Reaktionslösung wurde unter Zuführung von Sauerstoff im Kreis gepumpt und vor dem (Wieder-)Eintritt in das Absorberrohr gekühlt.

Der Spiegel konzentrierte die solare Einstrahlung in das Absorberrohr auf den Faktor 20.

Der Reaktor befand sich in Spanien (37.1° nördl. Breite).

Eine Lösung von 5.6 kg α-Thujen (GC-Gehalt 85%) und 5 g Bengalrosa in 27 kg Isopropanol wurde bei Sonnenschein unter kontinuierlicher Sauerstoffzugabe im Kreis gepumpt. Im Laufe der Reaktion wurden 4 g Bengalrosa nachdosiert. Nach 6 Stunden Reaktionszeit betrug der Umsatz 99 %. Eine Lösung aus 5.4 kg Natriumsulfit in 29 kg Wasser wurde zugegeben, die Mischung wurde auf 70°C erhitzt und 3 Stunden lang umgepumpt.

Aufarbeitung des Reaktionsgemisches: Bei Normaldruck wurden 32 kg Isopropanol/Wasser-Gemisch abdestilliert. Um die Phasentrennung zu erleichtern, wurde der Destillationssumpf mit 2 kg Toluol versetzt. Die sich abscheidende organische Phase wurde mit Wasser gewaschen und über 0,1 kg Na$_2$CO$_3$ destilliert. Man erhielt 4.5 kg 4-Hydroxy-β-thujen (GC-Gehalt 78 %) entsprechend einer Ausbeute von 65 % d. Th.

**Patentansprüche**

1. Verfahren zur Oxidation olefinischer Terpene mit Sauerstoff unter Lichteinfluß, dadurch gekennzeichnet, daß nicht mehr als 10 % der Gesamtenergie des emittierten Lichts auf den Wellenlängenbereich von 200 bis 400 nm, bezogen auf den von der Lichtquelle ausgestrahlten Wellenlängenbereich von 200 bis 700 nm, entfallen und die Bestrahlungsstärke so gewählt wird, daß die Reaktion in weniger als 12 Stunden einen Umsatz von 95 % erreicht.

2. Verfahren nach Anspruch 1, wonach man eine Bestrahlungsstärke von 5 bis 200 Sonnen einsetzt.

3. Verfahren nach Ansprüchen 1 und 2, wonach nicht mehr als 1 % der Gesamtenergie des emittierten Lichts auf den Wellenlängenbereich von 200 bis 300 nm, bezogen auf den von der Lichtquelle ausgestrahlten Wellenlängenbereich von 200 bis 700 nm, entfallen.

4. Verfahren zur Herstellung von Terpenalkoholen, wonach man olefinische Terpene gemäß dem Verfahren nach Ansprüchen 1 - 3 zu Terpen-Hydroperoxiden oxidiert und diese zu Terpenalkoholen reduziert.

5. Verfahren zur Herstellung von Rosenoxid, wonach man Citronellol gemäß dem Verfahren nach Ansprüchen 1 - 3 zu 3.7-Dimethyl-5-octen-1.7-diol oxidiert und dieses zu Rosenoxid cyclisiert.

6. Verfahren zur Herstellung von Sabinenhydrat, wonach man α-Thujen gemäß dem Verfahren nach Ansprüchen 1 - 3 oxidiert, das entstandene Hydroperoxid zu 4-Hydroxy-β-thujen reduziert und dieses zu Sabinenhydrat hydriert.

7. Verfahren zur Herstellung von Neroloxid, wonach man Nerol gemäß dem Verfahren nach Ansprüchen 1 - 3 oxidiert, das entstandene Hydroperoxid zu 3.7-Dimethyl-2.5-octadien-1.7-diol reduziert und dieses zu Neroloxid cyclisiert.

8. Verfahren zur Herstellung von Terpineol-(4), wonach man Terpinolen gemäß dem Verfahren nach Ansprüchen 1 - 3 oxidiert, das entstandene Hydroperoxid zu p-Mentha-1.8-dion-4-ol reduziert und dieses zu Terpineol-(4) hydriert.

**Claims**

1. Process for the oxidation of olefinic terpenes with oxygen under the influence of light, characterized in that no more than 10 % of the total energy of the light emitted is apportioned to the wavelength range of 200 to 400 nm, based on the wavelength range of 200 to 700 nm radiated by the light source, and the irradiation intensity is chosen so that the reaction achieves a conversion rate of 95 % in less than 12 hours.

2. Process according to Claim 1, wherein an irradiation intensity of 5 to 200 suns is used.

3. Process according to Claims 1 and 2, wherein no more than 1 % of the total energy of the emitted light is apportioned to the wavelength range of 200 to 300 nm, based on the wavelength range of 200 to 700 nm radiated by the light source.

4. Process for the preparation of terpene alcohols, wherein olefinic terpenes, in accordance with the process according to Claims 1 - 3, are oxidized to terpene hydroperoxides and these are reduced to terpene alcohols.

5. Process for the preparation of rose oxide, wherein citronellol, in accordance with the process according to Claims 1 - 3, is oxidized to 3,7-dimethyl-5-octene-1,7-diol, and this is cyclized to give rose oxide.

6. Process for the preparation of sabinene hydrate, wherein α-thujene, in accordance with the process according to Claims 1 - 3, is oxidized, the resulting hydroperoxide is reduced to 4-hydroxy-β-thujene and this is hydrogenated to give sabinene hydrate.

7. Process for the preparation of nerol oxide, wherein nerol, in accordance with the process according to Claims 1 - 3, is oxidized, the resulting hydroperoxide is reduced to 3,7-dimethyl-2,5-octadiene-1,7-diol and this is cyclized to give nerol oxide.

8. Process for the preparation of 4-terpineol, wherein terpinols, in accordance with the process according to Claims 1 - 3, are oxidized, the resulting hydroperoxide is reduced to p-mentha-1,8-dien-4-ol, and this is hydrogenated to give 4-terpineol.

**Revendications**

1. Procédé pour l'oxydation des terpènes oléfiniques par l'oxygène sous l'action de la lumière, caractérisé en ce que pas plus de 10 % de l'énergie totale de la lumière émise relève de l'intervalle des longueurs d'onde de 200 à 400 nm, par rapport à l'intervalle des longueurs d'onde émises par la source de lumière, de 200 à 700 nm, et on opère sous une intensité d'irradiation telle que la réaction atteigne un taux de conversion de 95 % en moins de 12 h.

2. Procédé selon la revendication 1, selon lequel on opère sous une intensité d'irradiation représentant de 5 à 200 fois l'intensité de la lumière solaire.

3. Procédé selon les revendications 1 et 2, selon lequel pas plus de 1 % de l'énergie totale de la lumière émise relève de l'intervalle des longueurs d'onde de 200 à 300 nm par rapport à l'intervalle des longueurs d'onde émises par la source de lumière, de 200 à 700 nm.

4. Procédé pour la préparation d'alcools terpéniques, selon lequel on oxyde des terpènes oléfiniques par le procédé de la revendication 1 en hydroperoxydes de terpènes que l'on réduit en alcools terpéniques.

5. Procédé de préparation de l'oxyde de rose selon lequel on oxyde le citronellol par le procédé de la revendication 1 en le 3,7-diméthyl-5-octène-1,7-diol qu'on cyclise en l'oxyde de rose.

6. Procédé pour la préparation de l'hydrate de sabinène selon lequel on oxyde l'α-thuyène par le procédé de la revendication 1, on réduit l'hydroperoxyde formé en le 4-hydroxy-β-thuyène et on hydrogène ce dernier en l'hydrate de sabinène.

7. Procédé pour la préparation de l'oxyde de nérol selon lequel on oxyde le nérol par le procédé de la revendication 1, on réduit l'hydroperoxyde formé en le 3,7-diméthyl-2,5-octadiène-1,7-diol et on cyclise ce dernier en l'oxyde de nérol.

8. Procédé pour la préparation du terpinéol-(4), selon lequel on oxyde le terpinolène par le procédé de la revendication 1, on réduit l'hydroperoxyde formé en le p-mentha-1,8-dione-4-ol et on hydrogène ce dernier en terpinéol(4).